# EUROPÄISCHE PATENTANMELDUNG

(11) **EP 4 623 688 A1**
(43) Veröffentlichungstag der Anmeldung: **01.10.2025**
(21) Anmeldenummer: 24166184.2
(22) Anmeldetag: 26.03.2024
(51) Int. Cl.: A01N 25/34, A01N 31/02, A01N 37/44, A01P 1/00, B65B 55/00, E03D 9/00

(54) **WISCHTUCH MIT LYOCELL-FASERN UND EINEM NIEDRIGALKOHOLISCHEN DESINFEKTIONSMITTEL**

(71) Anmelder: Bode Chemie GmbH, 22525 Hamburg (DE)
(72) Erfinder: LINKE, Christoph, 22525 Hamburg (DE); NODERER, Christina, 22525 Hamburg (DE); HARMS, Lena, 22525 Hamburg (DE); RODRIGUEZ-DIAZ, Javiera, 22525 Hamburg (DE)
(74) Vertreter: Paul Hartmann AG Patents & Licensing

(57) **Zusammenfassung**

Die vorliegende Erfindung betrifft ein Wischtuch für die Flächendesinfektion umfassend ein Substrat und ein flüssiges niedrigalkoholisches Desinfektionsmittel. Das Substrat umfasst Lyocell-Faser. Dadurch ist es nachhaltig und sehr gut kompatibel mit dem Desinfektionsmittel.

## Beschreibung

Die vorliegende Erfindung betrifft ein Wischtuch für die Flächendesinfektion umfassend ein Substrat und ein flüssiges Desinfektionsmittel. Die Erfindung betrifft zudem einen Spender, eine Verwendung sowie ein Herstellungsverfahren für das zuvor genannte Wischtuch.

Wischtücher zur Flächendesinfektion umfassend vollsynthetische Fasern wie Fasern aus Polyethylenterephthalat (PET) sind im Stand der Technik bekannt, zum Beispiel aus der EP 1 661 586 B1. Derartige Wischtücher können mit sehr vielen Desinfektionsmitteln kompatibel sein. Allerdings sind vollsynthetische Fasern beziehungsweise Kunststofffasern in der Regel nicht biologisch abbaubar und somit weniger nachhaltig.

Ebenso sind alkoholische Flächendesinfektionsmittel mit einer niedrigen Gesamtalkoholmenge von zum Bespiel nur etwa 30 Gew.-% im Stand der Technik bekannt. Diese können eine hohe Materialverträglichkeit aufweisen und daher auch an empfindlichen Oberflächen angewandt werden.

Die Aufgabe der vorliegenden Erfindung bestand darin, ein vorteilhaftes Wischtuch für die Flächendesinfektion anzugeben. Insbesondere sollte ein nachhaltiges Wischtuch für ein niedrigalkoholisches Flächendesinfektionsmittel gefunden werden. Diese Aufgabe wurde mit einem Wischtuch nach Anspruch 1 und 18, einem Spender nach Anspruch 15, einer Verwendung nach Anspruch 16 sowie einem Verfahren nach Anspruch 17 gelöst.

Erfindungsgemäß umfasst das vorliegende Wischtuch ein Substrat und ein flüssiges Desinfektionsmittel. Dabei bildet das Substrat das Tuchmaterial des Wischtuches, welches mit dem flüssigen Desinfektionsmittel getränkt ist. Die Tränkung des Substrats mit dem Desinfektionsmittel kann teilweise oder vollständig, bevorzugt nur teilweise, erfolgen. Das Wischtuch ist dadurch gekennzeichnet, dass das Substrat Lyocell-Fasern umfasst. Weiterhin ist die vorliegende Erfindung durch eine spezielle Zusammensetzung des Desinfektionsmittels gekennzeichnet, wonach dieses die nachfolgenden Komponenten umfasst:
i. 20 bis 50 Gew.-% mindestens eines einwertigen Alkohols mit 2 bis 4 Kohlenstoffatomen,
ii. mindestens eine weitere, antimikrobiell wirksame Substanz,
iii. optional mindestens einen Hilfsstoff, und
iv. Wasser.

Die in dem Desinfektionsmittel enthaltenen einwertigen Alkohole sind aliphatische Alkohole, sogenannte Alkanole.

Die Konzentrationsangaben der einzelnen Komponenten des Desinfektionsmittels beziehen sich im vorliegenden Dokument insofern nicht anders angegeben auf die Gesamtmenge beziehungsweise das Gesamtgewicht des Desinfektionsmittels. Weiterhin beziehen sich die Konzentrationsangaben im Hinblick auf die Stoffgruppe der einwertigen Alkohole mit 2 bis 4 Kohlenstoffatomen, die Stoffgruppe der weiteren antimikrobiell wirksamen Substanz und die Stoffgruppe der Hilfsstoffe im vorliegenden Dokument insofern nicht anders angegeben auf die Summe aller in dem Desinfektionsmittel enthaltenen einwertigen Alkohole mit 2 bis 4 Kohlenstoffatomen, weiteren antimikrobiell wirksamen Substanzen beziehungsweise Hilfsstoffe.

Lyocell-Fasern sind Regeneratfasern und bestehen aus dem nachwachsenden, pflanzlichen Rohstoff Cellulose. Sie sind biologisch abbaubar, nachhaltig und können Flüssigkeit gut aufnehmen. Durch die verwendeten Lyocell-Fasern ist das Substrat des vorliegenden Wischtuchs also nachhaltig. Durch die verwendeten Lyocell-Fasern kann das Substrat auch viel Flüssigkeit absorbieren. Wie die nachfolgenden Versuchsergebnisse noch zeigen, hat sich zudem herausgestellt, dass Lyocell-Fasern besonders gut für die vorliegend vorgesehenen Desinfektionsmittel mit einem niedrigen Alkoholgehalt geeignet sind. So hat sich in Versuchen gezeigt, dass die Lyocell-Fasern die Wirksamkeit eines solchen Desinfektionsmittels weniger stark beeinträchtigen als andere Cellulose-basierte Fasermaterialien. Das Substrat des vorliegenden Wischtuchs ist damit besonders gut mit den vorliegend vorgesehenen niedrigalkoholischen Desinfektionsmitteln kompatibel. Darüber hinaus kann das vorliegende Wischtuch aufgrund der vorgesehenen geringen Alkoholkonzentration im Desinfektionsmittel auch an empfindlichen Oberflächen angewandt werden. Weitere Vorteile des Wischtuchs ergeben sich aus den nachfolgend beschriebenen bevorzugten Ausführungsformen der Erfindung.

Typischerweise besteht das Wischtuch aus dem Substrat und dem Desinfektionsmittel. Ferner kann das Wischtuch einen Tränkungsgrad von 200 bis 600 Gew.-%, bevorzugt von 300 bis 500 Gew.-%, mehr bevorzugt von 350 bis 450 Gew.-% und besonders bevorzugt von etwa 400 Gew.-% aufweisen, bezogen auf das Gewicht des trockenen Substrats. Der Tränkungsgrad gibt das Massenverhältnis von Desinfektionsmittel zu Tuchgewicht an. Er kann berechnet werden, indem das Gewicht des trockenen Substrats von dem Gewicht des feuchten beziehungsweise getränkten Substrats abgezogen wird. Das Ergebnis (welches der Menge an Desinfektionsmittel in dem Wischtuch entspricht) wird dann durch das Gewicht des trockenen Substrats geteilt und schließlich der dabei erhaltene Wert mit 100 multipliziert, um den prozentualen Tränkungsgrad zu erhalten.

Das Wischtuch kann so ausgebildet sein, dass das aus ihm ausgepresste Desinfektionsmittel einen Reduktionsfaktor von mindestens 2, bevorzugt von mindestens 3 und besonders bevorzugt von mindestens 4 gegen Adenovirus aufweist (Reduktion um mindestens 2, 3 beziehungsweise 4 log-Stufen). Dabei kann der Reduktionsfaktor eine Obergrenze von 5 aufweisen, so dass das ausgepresste Desinfektionsmittel einen Reduktionsfaktor von 2 bis 5, bevorzugt von 3 bis 5 und besonders bevorzugt von 4 bis 5 gegen Adenovirus aufweisen kann. Die Prüfung der Wirksamkeit beziehungsweise die Bestimmung des Reduktionsfaktors kann wie im vorliegenden Dokument beschrieben erfolgen (siehe Versuchsergebnisse, 1. Punkt oder 3. Punkt). Somit kann der Reduktionsfaktor nach EN 14476 bei einer Kontaktzeit von 60 min, einer Prüfkonzentration von 80 % und einer hohen Belastung bestimmt werden. Alternativ kann der Reduktionsfaktor nach EN 14476 bei einer Kontaktzeit von 30 min, einer Prüfkonzentration von 97 %, einer hohen Belastung und Large-Volume-Plating (LVP) bestimmt werden.

Darüber hinaus kann das Wischtuch eine Reichweite von mindestens 1,5 m², bevorzugt von 1,5 bis 4 m² und insbesondere von 1,5 bis 3,5 m² aufweisen. Die Reichweite ist die Fläche, die mit einem getränkten Tuch vollständig benetzbar ist. Sie kann nach DIN SPEC 13285 bestimmt werden.

Wie bereits erwähnt umfasst das vorliegende Wischtuch ein Substrat, welches das Tuchmaterial des Wischtuches bildet und als Träger für das Desinfektionsmittel dient. Hierfür ist das Substrat absorbierend ausgebildet. Bevorzugt ist das Substrat ein Vliesstoff, insbesondere ein Spunlace-Vliesstoff. Nachfolgend werden vorteilhafte Parametermerkmale für das Substrat vorgeschlagen.

Das Substrat kann eine Dicke von 0,2 bis 0,8 mm, bevorzugt von 0,4 bis 0,6 mm und besonders bevorzugt von etwa 0,5 mm aufweisen. Dabei kann die Dicke des Substrats nach NWSP 120.6.R0 bestimmt werden (Dicke T1 bei einer Belastung von 0,005 N/cm²). Weiterhin kann das Substrat ein Flächengewicht von 30 bis 70 g/m², bevorzugt von 40 bis 60 g/m², mehr bevorzugt von 45 bis 55 g/m² und besonders bevorzugt von etwa 50 g/m² aufweisen. Dabei kann das Flächengewicht nach NWSP 130.1.R0 bestimmt werden. Zudem kann das Substrat eine Absorptionskapazität von 500 bis 1000 %, bevorzugt von 700 bis 900 %, mehr bevorzugt von 800 bis 900 % und besonders bevorzugt von 850 bis 880 % aufweisen. Dabei kann die Absorptionskapazität nach DIN 53923 : 2022-07 bestimmt werden. Mit derart hohen (maximalen) Absorptionskapazitäten können auch größere Mengen Desinfektionsmittel in das Substrat eingebracht werden, so dass mit einem Wischtuch eine größere Fläche gereinigt und desinfiziert werden kann.

Die genannten Werte für die Dicke, das Flächengewicht und die Absorptionskapazität beziehen sich auf das trockene Substrat.

Bezüglich seiner Form kann das Substrat insbesondere im Wesentlichen rechteckig ausgestaltet sein. Bezüglich seiner Größe kann das Substrat zum Beispiel eine Länge von 18 bis 22 cm, bevorzugt von etwa 20 cm, und eine Breite von 16 bis 20 cm, bevorzugt von etwa 17 oder 18 cm, aufweisen.

In einer besonders bevorzugten Ausführungsform der Erfindung besteht das Substrat aus Lyocell-Fasern. Das Substrat wird dann also ausschließlich von Lyocell-Fasern gebildet. Bei den vorliegend verwendeten Lyocell-Fasern handelt es sich normalerweise um konventionelle Lyocell-Fasern. Die Lyocell-Fasern sind also normalerweise nicht weiter modifiziert, insbesondere nicht weiter chemisch modifiziert, um beispielsweise ihre Ladung oder sonstige Eigenschaften zu verändern.

Nachfolgend wird näher auf das Desinfektionsmittel eingegangen, mit dem das Substrat vorgetränkt wird. Im Hinblick auf die zuvor genannte Komponente i., also dem mindestens einen einwertigen Alkohol mit 2 bis 4 Kohlenstoffatomen, kann das Desinfektionsmittel wie folgt beschaffen sein.

Das Desinfektionsmittel kann 20 bis 40 Gew.-%, bevorzugt 25 bis 35 Gew.-% und besonders bevorzugt 25 bis 32 Gew.-% des mindestens einen Alkohols enthalten.

Vorzugsweise ist der Alkohol beziehungsweise sind die Alkohole ausgewählt aus der Gruppe bestehend aus Ethanol, 1-Propanol und 2-Propanol (Isopropanol).

Das Desinfektionsmittel kann nur einen einzigen Alkohol enthalten, zum Beispiel nur Ethanol oder nur 2-Propanol. Ebenso kann das Desinfektionsmittel jedoch eine Mischung aus mindestens zwei, insbesondere aus drei Alkoholen enthalten. Eine Mischung von Alkoholen kann stärker antimikrobiell wirksam sein als ein einzelner Alkohol in gleicher Menge. Entsprechend kann das Desinfektionsmittel eine Mischung aus Ethanol, 1-Propanol und 2-Propanol enthalten. Dabei enthält das Desinfektionsmittel vorteilhafterweise mindestens 5 und höchstens 15 Gew.-% jeder einzelnen Alkoholkomponente. Insbesondere enthält das Desinfektionsmittel 13 bis 15 Gew.-% Ethanol, 5 bis 6,5 Gew.-% 1-Propanol und 9 bis 10,5 Gew.-% 2-Propanol. Es kann allgemein vorteilhaft sein, wenn das Desinfektionsmittel nicht mehr als 6,5 Gew.-% 1-Propanol, bevorzugt 5 bis 6,5 Gew.-% 1-Propanol, enthält.

Das Desinfektionsmittel kann 0,1 bis 3 Gew.-%, bevorzugt 0,1 bis 2 Gew.-% der mindestens einer weiteren antimikrobiell wirksamen Substanz enthalten. Die weitere antimikrobiell wirksame Substanz beziehungsweise die weiteren antimikrobiell wirksamen Substanzen können ausgewählt sein aus der Gruppe bestehend aus antimikrobiellen Tensiden, Phenoxyethanol und organischen Säuren. Bevorzugt ist die weitere antimikrobiell wirksame Substanz ein antimikrobielles Tensid, insbesondere ein antimikrobielles, amphoteres Tensid. Mit einer oder mehreren zusätzlichen antimikrobiell wirksamen Substanzen kann die antimikrobielle Wirksamkeit des Desinfektionsmittels vorteilhaft verstärkt werden.

Das Desinfektionsmittel kann 0,1 bis 2 Gew.-%, bevorzugt 0,1 bis 1 Gew.-% und besonders bevorzugt 0,1 bis 0,5 Gew.-% des antimikrobiellen, amphoteren Tensids enthalten. Vorzugsweise ist das antimikrobielle, amphotere Tensid N-Alkylaminopropylglycin, insbesondere N-Alkylaminopropylglycin mit Alkyl gleich C10 bis C16. N-Alkylaminopropylglycin ist auch unter der ECHA-Bezeichnung Amines, N-C10-16-alkyltrimethylenedi-, reaction products with chloroacetic acid bekannt (CAS-Nr. 139734-65-9).

Eine geeignete organische Säure als zusätzliche antimikrobiell wirksame Substanz für das vorliegende Desinfektionsmittel kann aus der Gruppe bestehend aus Ascorbinsäure, Benzoesäure, Essigsäure, Glykolsäure, Milchsäure, Propionsäure und Weinsäure ausgewählt werden. Es kann vorteilhafterweise auch eine Mischung von mehreren der zuvor genannten organischen Säuren in dem Desinfektionsmittel enthalten sein.

Wie bereits eingangs erwähnt, kann das Desinfektionsmittel optional mindestens einen Hilfsstoff enthalten. So kann das Desinfektionsmittel 0 bis 10 Gew.-%, 0 bis 5 Gew.-%, 0 bis 2 Gew.-%, 0 bis 1 Gew.-%, 0,01 bis 10 Gew.-%, 0,01 bis 5 Gew.-%, 0,01 bis 2 Gew.-% oder 0,01 bis 1 Gew.-% Hilfsstoff beziehungsweise Hilfsstoffe enthalten. Bevorzugt ist der Hilfsstoff beziehungsweise sind die Hilfsstoffe ausgewählt aus der Gruppe bestehend aus Farbstoffen, Duftstoffen (Parfüm), Lösungsmitteln, pH-Stellmitteln und Tensiden. Besonders bevorzugt ist der Hilfsstoff beziehungsweise sind die Hilfsstoffe ausgewählt aus der Gruppe bestehend aus pH-Stellmitteln und Tensiden. Es kann vorteilhaft sein, die Anzahl sowie die Konzentration der Hilfsstoffe im Desinfektionsmittel eher gering zu halten. Dadurch können Rückstände des Desinfektionsmittels auf den zu reinigenden und zu desinfizierenden Oberflächen vermieden oder zumindest reduziert werden. In diesem Sinne kann es gemäß einer Ausführungsform der Erfindung auch vorgesehen sein, dass das Desinfektionsmittel keine Hilfsstoffe enthält. Das zuvor beschriebene antimikrobielle, amphotere Tensid N-Alkylaminopropylglycin sowie Phenoxyethanol werden vorliegend nicht als Hilfsstoffe verstanden.

Nachfolgend werden Mengenangaben und Beispiele für die einzelnen Hilfsstoffe vorgeschlagen:

| | |
|---|---|
| Farbstoffe: | 0,01 bis 5 Gew.-% |
| Duftstoffe (Parfüm): | 0,01 bis 5 Gew.-% |
| Lösungsmittel: | 0,1 bis 5 Gew.-%; zum Beispiel ein Ether wie 3-Methoxy-3-Methyl-1-Butanol (MMB), ein Glykolether wie Propylenglycol-n-butylether (PnB) |
| pH-Stellmittel: | anorganische Basen, anorganische Säuren und organische Säuren sowie Salze der organischen Säuren; zum Beispiel Kaliumhydroxid, Natriumhydroxid, Phosphorsäure, Salzsäure und Essigsäure sowie Salze der Essigsäure |
| Tenside: | 0,01 bis 5 Gew.-%; zum Beispiel Alkylphosphonate, Alkylpolyglucoside, Alkylsulfate, Alkylsulfonate und Glykolipide (wie Sophorolipid) |

In einer Ausführungsform der Erfindung ist das pH-Stellmittel eine anorganische Base oder eine anorganische Säure. So kann das pH-Stellmittel ausgewählt sein aus der Gruppe bestehend aus Kaliumhydroxid, Natriumhydroxid, Phosphorsäure und Salzsäure. In einer anderen Ausführungsform der Erfindung ist das Tensid (als Hilfsstoff) ausgewählt aus der Gruppe bestehend aus Alkylphosphonaten, Alkylpolyglucosiden, Alkylsulfaten, Alkylsulfonaten und Glykolipiden.

In der Regel weist das Desinfektionsmittel einen vergleichsweise hohen Wasseranteil auf. Zum Beispiel kann das Desinfektionsmittel 40 bis 80 Gew.-%, bevorzugt 50 bis 80 Gew.-%, besonders bevorzugt 60 bis 80 Gew.-% und insbesondere 65 bis 75 Gew.-% Wasser enthalten. Zudem kann das Desinfektionsmittel einen pH-Wert von weniger als 10, bevorzugt von 7 bis 9, mehr bevorzugt von 7,5 bis 8,5 und besonders bevorzugt von etwa 8,0 aufweisen. Dabei wird insbesondere auf eine Ausführungsform der Erfindung abgestellt, in der keine organischen Säuren als Wirkverstärker vorgesehen sind.

Es kann vorgesehen sein, dass das Desinfektionsmittel abgesehen von dem Alkohol beziehungsweise den Alkoholen und den zuvor genannten antimikrobiell wirksamen Substanzen (nämlich die antimikrobiellen Tenside, Phenoxyethanol sowie die organischen Säuren) im Wesentlichen keine weitere antimikrobiell wirksame Komponente enthält. Ebenso kann vorgesehen sein, dass das Desinfektionsmittel keine antimikrobiellen Wirkstoffe ausgewählt aus der Gruppe bestehend aus quartären Ammoniumverbindungen, Aldehyden, Guanidinen, Aktivchlor, Aminen (außer N-Alkylaminopropylglycin), Sauerstoffabspaltern und aromatischen Alkoholen enthält.

Nachfolgend werden beispielhaft verschiedene Zusammensetzungen des Desinfektionsmittels angegeben.

In einer Ausführungsform der Erfindung umfasst das Desinfektionsmittel die folgenden Komponenten:
i. 20 bis 50 Gew.-% mindestens eines einwertigen Alkohols mit 2 bis 4 Kohlenstoffatomen,
ii. 0,1 bis 3 Gew.-% mindestens einer weiteren, antimikrobiell wirksamen Substanz ausgewählt aus der Gruppe bestehend aus antimikrobiellen Tensiden, Phenoxyethanol und organischen Säuren,
iii. optional mindestens einen Hilfsstoff, und
iv. Wasser.

In einer Ausführungsform der Erfindung umfasst das Desinfektionsmittel die folgenden Komponenten:
i. 20 bis 50 Gew.-% mindestens eines einwertigen Alkohols mit 2 bis 4 Kohlenstoffatomen,
ii. 0,1 bis 3 Gew.-% mindestens einer weiteren, antimikrobiell wirksamen Substanz ausgewählt aus der Gruppe bestehend aus N-Alkylaminopropylglycin, Phenoxyethanol, Ascorbinsäure, Benzoesäure, Essigsäure, Glykolsäure, Milchsäure, Propionsäure und Weinsäure,
iii. optional mindestens einen Hilfsstoff, und
iv. Wasser.

In einer bevorzugten Ausführungsform der Erfindung umfasst das Desinfektionsmittel die folgenden Komponenten:
i. 25 bis 32 Gew.-% einer Mischung aus Ethanol, 1-Propanol und 2-Propanol, wobei mindestens 5 und höchstens 15 Gew.-% jeder einzelnen Alkoholkomponente enthalten sind,
ii. 0,1 bis 2,0 Gew.-% eines antimikrobiellen, amphoteren Tensids, bevorzugt N-Alkylaminopropylglycin, insbesondere N-Alkylaminopropylglycin mit Alkyl gleich C10 bis C16,
iii. 0 bis 10 Gew.-% mindestens eines Hilfsstoffs, bevorzugt keinen Hilfsstoff, und
iv. 65 bis 75 Gew.-% Wasser.

In einer bevorzugten Ausführungsform der Erfindung besteht das Desinfektionsmittel aus den folgenden Komponenten:
i. 25 bis 32 Gew.-% einer Mischung aus Ethanol, 1-Propanol und 2-Propanol, wobei mindestens 5 und höchstens 15 Gew.-% jeder einzelnen Alkoholkomponente enthalten sind,
ii. 0,1 bis 2,0 Gew.-% eines antimikrobiellen, amphoteren Tensids, bevorzugt N-Alkylaminopropylglycin, insbesondere N-Alkylaminopropylglycin mit Alkyl gleich C10 bis C16, und
iii. Wasser,
wobei sich die Komponenten zu 100 Gew.-% ergänzen.

Ein weiterer Gegenstand der vorliegenden Erfindung ist ein Spender. Dieser umfasst ein oder mehrere Wischtücher wie vorangehend beschrieben. Der Spender kann von einem im Wesentlichen starren, eimerartigen Behälter oder Standbodenbeutel mit einer verschließbaren Entnahmeöffnung gebildet sein, welcher in seinem Inneren eine Rolle mit den Wischtüchern enthält. Alternativ kann der Spender auch von einer flexiblen Folienverpackung mit einer verschließbaren Entnahmeöffnung gebildet sein, welche einen Stapel der Wischtücher umhüllt (Schlauchbeutelverpackung beziehungsweise Flowpack-Verpackung). Der Spender, insbesondere der als Flowpack ausgebildete Spender, kann einen Stapel von 60 bis 200, bevorzugt von 80 bis 200, mehr bevorzugt von 120 bis 200 und besonders bevorzugt von 120 bis 160 Wischtüchern enthalten.

Zudem wird die Verwendung eines Wischtuches wie vorangehend beschrieben zur Reinigung und/oder Desinfektion einer unbelebten Oberfläche beansprucht. Bei der Oberfläche kann es sich beispielsweise um eine Oberfläche eines Gerätes oder eine Oberfläche eines Einrichtungsgegenstandes insbesondere im medizinischen Bereich handeln. Aufgrund der hohen Materialverträglichkeit des vorliegenden Desinfektionsmittels kann es sich bei der Oberfläche sogar um eine Bildschirmoberfläche eines elektronischen Gerätes handeln.

Es wird auch ein Verfahren zur Herstellung eines Wischtuches wie vorangehend beschrieben angegeben. Das Verfahren umfasst die folgenden Schritte:
a. Bereitstellen eines Substrats umfassend Lyocell-Fasern,
b. Bereitstellen eines flüssigen Desinfektionsmittels umfassend die Komponenten:
   i. 20 bis 50 Gew.-% mindestens eines einwertigen Alkohols mit 2 bis 4 Kohlenstoffatomen,
   ii. mindestens eine weitere antimikrobiell wirksame Substanz,
   iii. optional mindestens einen Hilfsstoff, und
   iv. Wasser,
c. Tränken des Substrats mit dem Desinfektionsmittel. Dabei ändert sich der pH-Wert des Desinfektionsmittels bevorzugt um weniger als 10 %, insbesondere um weniger als 5 %. Die Änderung des pH-Werts bezieht sich auf den pH-Wert des Desinfektionsmittels bevor es mit dem Substrat in Kontakt gebracht wird und kann sowohl eine Erhöhung als auch eine Verringerung des pH-Werts sein. Um die Änderung des pH-Werts zu ermitteln, wird entsprechend der pH-Wert des Desinfektionsmittels vor und nach dem Tränken des Substrats mit dem Desinfektionsmittel bestimmt. Der pH-Wert nach dem Tränken kann bestimmt werden, indem das Desinfektionsmittel mindestens 72 Stunden nach dem Tränken aus dem Wischtuch gepresst und vermessen wird (siehe auch Versuchsergebnisse, 2. Punkt).

Das Wischtuch kann in all seinen vorangehend beschriebenen Ausführungsformen für den Spender und die Verwendung eingesetzt und mit dem Verfahren hergestellt werden. Die zusätzlichen Merkmale des Wischtuches in seinen speziellen Ausführungsformen sind daher ebenso im Zusammenhang mit dem Spender, der Verwendung sowie dem Verfahren offenbart.

Schließlich wird das Wischtuch in Anspruch 18 ohne zwingend eine weitere antimikrobiell wirksame Substanz im Desinfektionsmittel enthalten zu müssen unabhängig beansprucht.

Das Wischtuch nach Anspruch 18 umfasst ein Substrat und ein flüssiges Desinfektionsmittel und ist dadurch gekennzeichnet, dass
- das Substrat Lyocell-Fasern umfasst und
- das Desinfektionsmittel die folgenden Komponenten umfasst:
   i. 20 bis 50 Gew.-% mindestens eines einwertigen Alkohols mit 2 bis 4 Kohlenstoffatomen,
   ii. optional eine weitere antimikrobiell wirksame Substanz,
   iii. optional mindestens einen Hilfsstoff,
   iv. Wasser,
wobei das Wischtuch so ausgebildet ist, dass das aus ihm ausgepresste Desinfektionsmittel einen Reduktionsfaktor von mindestens 2, bevorzugt von mindestens 3 und besonders bevorzugt von mindestens 4 gegen Adenovirus aufweist (Reduktion um mindestens 2, 3 beziehungsweise 4 log-Stufen). Dabei kann der Reduktionsfaktor eine Obergrenze von 5 aufweisen, so dass das ausgepresste Desinfektionsmittel einen Reduktionsfaktor von 2 bis 5, bevorzugt von 3 bis 5 und besonders bevorzugt von 4 bis 5 gegen Adenovirus aufweisen kann. Die Prüfung der Wirksamkeit beziehungsweise die Bestimmung des Reduktionsfaktors kann wie im vorliegenden Dokument beschrieben erfolgen (siehe Versuchsergebnisse, 1. Punkt oder 3. Punkt). Somit kann der Reduktionsfaktor nach EN 14476 bei einer Kontaktzeit von 60 min, einer Prüfkonzentration von 80 % und einer hohen Belastung bestimmt werden. Alternativ kann der Reduktionsfaktor nach EN 14476 bei einer Kontaktzeit von 30 min, einer Prüfkonzentration von 97 %, einer hohen Belastung und Large-Volume-Plating (LVP) bestimmt werden.

Die Merkmale des Wischtuches nach Anspruch 1 in all seinen vorangehend beschriebenen Ausführungsformen können einzeln oder in beliebiger Kombination auf das Wischtuch nach Anspruch 18 übertragen werden.

### Ausführungsbeispiel

Ein erfindungsgemäßes Wischtuch in einer bevorzugten Ausführungsform kann folgendermaßen erhalten werden.
a. Es wird ein Vliestuch (anspruchsgemäßes Substrat) bestehend aus Lyocell-Fasern bereitgestellt. Das Vliestuch kann rechteckig mit Abmessungen von 20 cm x 18 cm (Länge x Breite) ausgebildet sein und ein Flächengewicht (Grammatur) von 50 g/m² aufweisen. Ein einzelnes Tuch wiegt dann 1,8 g. Ein solches Vlies ist zum Beispiel Sawatex^{®} 2618/2018 der Fa. Sandler.
b. Es wird ein niedrigalkoholisches Desinfektionsmittel bereitgestellt, welches aus den folgenden Komponenten besteht:
   i. 14 Gew.-% Ethanol,
   ii. 6 Gew.-% 1-Propanol
   iii. 10 Gew.-% 2-Propanol
   iv. 0,2 Gew.-% N-Alkylaminopropylglycin
   v. Rest Wasser (also 69,8 Gew.-% Wasser)
c. Das Vliestuch wird mit dem Desinfektionsmittel getränkt. Der Tränkungsgrad kann 400 % betragen. Bei dem zuvor genannten Vliestuch entspricht dies einer Menge von 7,2 g Desinfektionsmittel pro Tuch. Ein solches Vliestuch kann mit den zuvor genannten Werten für die Größe (20 cm x 18 cm), dem Flächengewicht (50 g/m²) und dem Tränkungsgrad (400 %) eine Reichweite von mindestens 1,5 m² aufweisen (bestimmt nach DIN SPEC 13285).
d. Mehrere der getränkten Vliestücher, zum Beispiel 80 oder bevorzugt 120 getränkte Vliestücher, werden gestapelt und in Verbundfolie als Schlauchbeutelverpackung (Flowpack) mit einer verschließbaren Entnahmeöffnung verpackt. Dabei wird ein erfindungsgemäßer Spender in einer bevorzugten Ausführungsform erhalten.

### Versuchsergebnisse

### 1. Einfluss von cellulosischen Fasern auf die Wirksamkeit eines niedrigalkoholischen Desinfektionsmittels

Um den Einfluss von cellulosischen Tuchfasern auf die Wirksamkeit eines niedrigalkoholischen Desinfektionsmittels zu untersuchen, wurde wie folgt vorgegangen. Es wurde ein Ansatz eines Desinfektionsmittels bestehend aus 30 Gew.-% Alkohol, Wasser und einem pH-Stellmittel hergestellt und auf vier unterschiedliche Vliestücher (20 cm x 18 cm, 50 g/m², 80 Stück verpackt in Kunststoffverbundfolie) gegeben. Die Kontaktzeit zwischen Vliestuch und Desinfektionsmittel betrug mindestens 72 h bei Raumtemperatur. Der Tränkungsgrad aller Vliestücher war mit 400 Gew.-% identisch. Alle Vliestücher wurden zum gleichen Zeitpunkt entnommen und das enthaltende Desinfektionsmittel ausgepresst (Proben B bis E). Zusätzlich wurde das Desinfektionsmittel ohne Tuchkontakt als Referenz untersucht (Probe A). In den nachfolgenden Tabellen 1 und 2 sind die Zusammensetzung des verwendeten Desinfektionsmittels sowie der verwendeten Vliestücher angegeben.

**Tabelle 1: Zusammensetzung des verwendeten Desinfektionsmittels**

| **Komponente** | **Menge (Gew.-%)** |
|---|---|
| Ethanol | 14,0 % |
| 1-Propanol | 6,0 % |
| 2-Propanol | 10,0 % |
| Wasser | ad 100 % |
| pH-Stellmittel (Acetat / Essigsäure) | ad pH 8,0 |

**Tabelle 2: Zusammensetzung der verwendeten Vliestücher und die Bezeichnung der aus den Vliestüchern ausgepressten Proben**

| **Tuch-Nr.** | **Zusammensetzung** | **Probenbezeichnung** |
|---|---|---|
| 1 | 100 % Polyester-Fasern (PET) | B |
| 2 | Mischung aus Viskose und Pulpe (Carded-Airlaid-Carded, CAC) | C |
| 3 | 100 % Viskose-Fasern | D |
| 4 | 100 % Lyocell-Fasern | E |

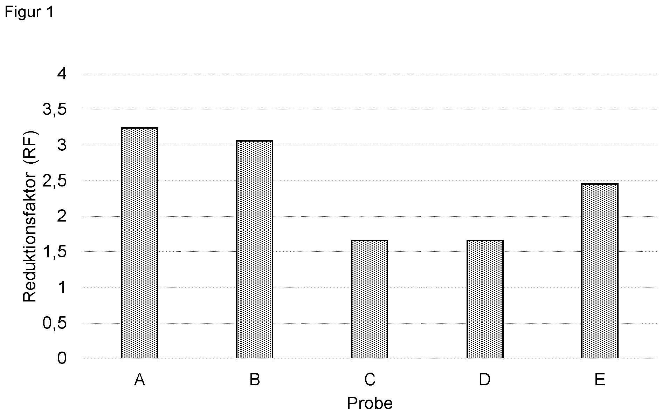

Die Proben-Lösungen A bis E wurden dann im Hinblick auf ihre Wirksamkeit gegen unbehüllte Viren geprüft. Um selbst eine geringe Beeinflussung durch das Tuchmaterial zu erfassen, wurde eine Suspensionsprüfung gemäß EN 14476 an Adenovirus durchgeführt (Kontaktzeit 60 min, 80 % Prüfkonzentration, hohe Belastung). Adenoviren sind wirksamkeitslimitierend bei niedrigalkoholischen Produkten, so dass sich hier bereits eine geringe Beeinflussung durch das Tuchmaterial im Reduktionsfaktor (RF) bemerkbar macht. Zudem werden durch die Suspensionsprüfung etwaige mechanische Einflüsse bedingt durch unterschiedliche Tuchstrukturen anders als beispielsweise im 4-Felder-Test ausgeschlossen oder zumindest reduziert. Die Ergebnisse des antimikrobiellen Tests sind in Tabelle 3 sowie ergänzend in Figur 1 als Balkendiagramm dargestellt.

**Tabelle 3: Ergebnisse des antimikrobiellen Tests (Suspensionsprüfung gem. EN 14476 an Adenovirus)**

| **Probe** | **A** | **B** | **C** | **D** | **E** |
|---|---|---|---|---|---|
| Tuch | keines (Referenz) | 1 (PET-Vlies) | 2 (CAC-Vlies) | 3 (Viskose-Vlies | 4 (Lyocell-Vlies) |
| Reduktionsfaktor (RF) | 3,23 | 3,06 | 1,66 | 1,66 | 2,45 |

Die erhaltenen Reduktionsfaktoren zeigen, dass alle getesteten Vliestücher die antimikrobielle Wirksamkeit des Desinfektionsmittels verringert haben (erkennbar an den niedrigeren RF-Werten im Vergleich zur Kontroll-beziehungsweise Referenzprobe A). Dieser negative Effekt auf die Aktivität des Desinfektionsmittels war bei den cellulose-basierten Vliestüchern 2 bis 4 stärker ausgeprägt als bei dem synthetischen PET-Vliestuch 1. Zudem zeigen die Ergebnisse interessanterweise deutliche Unterschiede innerhalb der Gruppe der cellulose-basierten Vliestücher. So hat das Vliestuch 4 aus Lyocell die Wirksamkeit des Desinfektionsmittels überraschenderweise deutlich weniger beeinträchtigt als die beiden Vliestücher 2 und 3 aus Zellstoff und/oder Viskose. Somit sind Lyocell-Fasern diesen Versuchsergebnissen zufolge die mit Abstand beste Wahl, um ein nachhaltiges Wischtuch für ein niedrigalkoholisches Desinfektionsmittel herzustellen.

### 2. Einfluss von cellulosischen Fasern auf den pH-Wert und das Aussehen eines niedrigalkoholischen Desinfektionsmittels

Die Vliestücher 1 bis 4 aus Tabelle 2 wurden mit dem Desinfektionsmittel aus Tabelle 1 (eingestellt auf einen pH-Wert von 7,9) wie zuvor in Punkt 1 beschrieben behandelt. Der pH-Wert und das Aussehen (Färbung) der ausgepressten Proben G bis J wurde dann bestimmt. **Tabelle 4** enthält die Ergebnisse der pH-Messungen und der optischen Prüfungen.

**Tabelle 4: pH-Messungen und optische Prüfungen mit 400 % Tränkung bei Proben G bis J**

| **Probe** | **F** | **G** | **H** | **I** | **J** |
|---|---|---|---|---|---|
| Tuch | keines (Referenz) | 1 (PET-Vlies) | 2 (CAC-Vlies) | 3 (Viskose-Vlies | 4 (Lyocell-Vlies) |
| pH-Wert | 7,9 | 7,9 | 7,0 | 7,0 | 7,7 |
| Aussehen/ Färbung | klare Lösung (farblos) | klare Lösung (farblos) | gelblich | schwach gelblich | klare Lösung (farblos) |

Ergänzend wurden die Vliestücher 1 bis 4 aus Tabelle 2 mit dem Desinfektionsmittel aus Tabelle 1 (eingestellt auf einen pH-Wert von 7,9) ähnlich wie zuvor in Punkt 1 beschrieben behandelt, wobei diesmal ein niedrigerer Tränkungsgrad von 320 Gew.-% verwendet wurde. Der pH-Wert und das Aussehen (Färbung) der ausgepressten Proben K bis N im Vergleich zur Referenz-Probe F wurde dann bestimmt und ist in **Tabelle 5** dargestellt.

**Tabelle 5: pH-Messungen und optische Prüfungen mit 320 % Tränkung bei Proben K bis N**

| **Probe** | **F** | **K** | **L** | **M** | **N** |
|---|---|---|---|---|---|
| Tuch | keines (Referenz) | 1 (PET-Vlies) | 2 (CAC-Vlies) | 3 (Viskose-Vlies | 4 (Lyocell-Vlies) |
| pH-Wert | 7,9 | 7,8 | 6,5 | 6,7 | 7,5 |
| Aussehen/ Färbung | klare Lösung (farblos) | klare Lösung (farblos) | gelblich | schwach gelblich | klare Lösung (farblos) |

Die Ergebnisse zeigen, dass die cellulose-basierten Vliestücher sowohl den pH-Wert als auch das Aussehen des Desinfektionsmittels in einem unterschiedlichen Ausmaß beeinflussen. Bei den Lyocell-Fasern ist der Einfluss ebenso wie bei den PET-Fasern vorteilhafterweise nur gering, während das CAC-Vliestuch und das Viskose-Vliestuch den pH-Wert und das Aussehen des Desinfektionsmittels deutlich stärker verändern. Derartige Veränderungen im pH-Wert und Aussehen wie insbesondere bei den Proben H und I sowie L und M beobachtet sind allgemein unerwünscht und können auf eine Reaktion des Vliesmaterials mit dem Desinfektionsmittel hinweisen.

### 3. Test der Wirksamkeit eines erfindungsgemäßen Wischtuches in einer bevorzugten Ausführungsform gegen Adenovirus

Für den Test wurde das Desinfektionsmittel gemäß Tabelle 6 hergestellt und auf Vliestücher bestehend aus Lyocell-Fasern (20 cm x 18 cm, 50 g/m², 80 Stück verpackt in Kunststoffverbundfolie) gegeben. Die Kontaktzeit zwischen den Vliestüchern und dem Desinfektionsmittel betrug mindestens 72 h bei Raumtemperatur. Der Tränkungsgrad der Vliestücher mit dem Desinfektionsmittel betrug 400 Gew.-%. Mit dem aus den Vliestüchern ausgepressten Desinfektionsmittel wurde eine Suspensionsprüfung gemäß EN 14476 an Adenovirus unter den folgenden Bedingungen durchgeführt:
- Kontaktzeit 30 min
- 97 % Prüfkonzentration
- hohe Belastung
- Large-Volume-Plating (LVP)

Der Test hat gezeigt, dass das aus den untersuchten Wischtüchern ausgepresste Desinfektionsmittel einen Reduktionsfaktor von 4 gegen Adenovirus aufweisen kann.

**Tabelle 6: Zusammensetzung des verwendeten Desinfektionsmittels (pH 8)**

| **Komponente** | **Menge (Gew.-%)** |
|---|---|
| Ethanol | 14,0 % |
| 1-Propanol | 6,0 % |
| 2-Propanol | 10,0 % |
| N-Alkylaminopropylglycin | 0,2 % |
| Wasser | ad 100 % |

## Patentansprüche

1. Wischtuch für die Flächendesinfektion umfassend ein Substrat und ein flüssiges Desinfektionsmittel,
**dadurch gekennzeichnet, dass**
- das Substrat Lyocell-Fasern umfasst und
- das Desinfektionsmittel die folgenden Komponenten umfasst:
i. 20 bis 50 Gew.-% mindestens eines einwertigen Alkohols mit 2 bis 4 Kohlenstoffatomen,
ii. mindestens eine weitere antimikrobiell wirksame Substanz,
iii. optional mindestens einen Hilfsstoff,
iv. Wasser.

2. Wischtuch nach Anspruch 1, **dadurch gekennzeichnet, dass** das Wischtuch einen Tränkungsgrad von 200 bis 600 Gew.-%, bevorzugt von 300 bis 500 Gew.-%, mehr bevorzugt von 350 bis 450 Gew.-% und besonders bevorzugt von etwa 400 Gew.-% aufweist, bezogen auf das Gewicht des trockenen Substrats.

3. Wischtuch nach Anspruch 1 oder 2, **dadurch gekennzeichnet, dass** das Wischtuch so ausgebildet ist, dass das aus ihm ausgepresste Desinfektionsmittel einen Reduktionsfaktor von mindestens 2, bevorzugt von mindestens 3 und besonders bevorzugt von mindestens 4 gegen Adenovirus aufweist.

4. Wischtuch nach einem der vorangehenden Ansprüche, **dadurch gekennzeichnet, dass** das Wischtuch eine Reichweite von mindestens 1,5 m², bevorzugt von 1,5 bis 4 m² und insbesondere von 1,5 bis 3,5 m² aufweist.

5. Wischtuch nach einem der vorangehenden Ansprüche, **dadurch gekennzeichnet, dass** das Substrat ein Vliesstoff, insbesondere ein Spunlace-Vliesstoff, ist.

6. Wischtuch nach einem der vorangehenden Ansprüche, **dadurch gekennzeichnet, dass**
- das Substrat eine Dicke von 0,2 bis 0,8 mm, bevorzugt von 0,4 bis 0,6 mm und besonders bevorzugt von etwa 0,5 mm aufweist, und/oder
- das Substrat ein Flächengewicht von 30 bis 70 g/m², bevorzugt von 40 bis 60 g/m², mehr bevorzugt von 45 bis 55 g/m² und besonders bevorzugt von etwa 50 g/m² aufweist, und/oder
- das Substrat eine Absorptionskapazität von 500 bis 1000 %, bevorzugt von 700 bis 900 %, mehr bevorzugt von 800 bis 900 % und besonders bevorzugt von 850 bis 880 % aufweist.

7. Wischtuch nach einem der vorangehenden Ansprüche, **dadurch gekennzeichnet, dass** das Substrat aus Lyocell-Fasern besteht.

8. Wischtuch nach einem der vorangehenden Ansprüche, **dadurch gekennzeichnet, dass** das Desinfektionsmittel 20 bis 40 Gew.-%, bevorzugt 25 bis 35 Gew.-% und besonders bevorzugt 25 bis 32 Gew.-% des mindestens einen Alkohols enthält.

9. Wischtuch nach einem der vorangehenden Ansprüche, **dadurch gekennzeichnet, dass** der Alkohol ausgewählt ist aus der Gruppe bestehend aus Ethanol, 1-Propanol und 2-Propanol.

10. Wischtuch nach einem der vorangehenden Ansprüche, **dadurch gekennzeichnet, dass** das Desinfektionsmittel eine Mischung aus Ethanol, 1-Propanol und 2-Propanol enthält, wobei das Desinfektionsmittel bevorzugt mindestens 5 und höchstens 15 Gew.-% jeder einzelnen Alkoholkomponente enthält.

11. Wischtuch nach einem der vorangehenden Ansprüche, **dadurch gekennzeichnet, dass** das Desinfektionsmittel 0,1 bis 3 Gew.-%, bevorzugt 0,1 bis 2 Gew.-% der mindestens einen antimikrobiell wirksamen Substanz enthält.

12. Wischtuch nach einem der vorangehenden Ansprüche, **dadurch gekennzeichnet, dass** die antimikrobiell wirksame Substanz ausgewählt ist aus der Gruppe bestehend aus antimikrobiellen Tensiden, Phenoxyethanol und organischen Säuren, wobei die antimikrobiell wirksame Substanz bevorzugt ein antimikrobielles Tensid, insbesondere ein antimikrobielles, amphoteres Tensid, ist.

13. Wischtuch nach Anspruch 12, **dadurch gekennzeichnet, dass** das antimikrobielle, amphotere Tensid N-Alkylaminopropylglycin, insbesondere N-Alkylaminopropylglycin mit Alkyl gleich C10 bis C16, ist.

14. Wischtuch nach einem der vorangehenden Ansprüche, **dadurch gekennzeichnet, dass** das Desinfektionsmittel die folgenden Komponenten umfasst:
i. 25 bis 32 Gew.-% einer Mischung aus Ethanol, 1-Propanol und 2-Propanol, wobei mindestens 5 und höchstens 15 Gew.-% jeder einzelnen Alkoholkomponente enthalten sind,
ii. 0,1 bis 2,0 Gew.-% eines antimikrobiellen, amphoteren Tensids, bevorzugt N-Alkylaminopropylglycin, insbesondere N-Alkylaminopropylglycin mit Alkyl gleich C10 bis C16,
iii. 0 bis 10 Gew.-% mindestens eines Hilfsstoffs, bevorzugt keinen Hilfsstoff,
iv. 65 bis 75 Gew.-% Wasser.

15. Spender umfassend ein oder mehrere Wischtücher nach einem der vorangehenden Ansprüche, wobei der Spender bevorzugt einen Stapel von 60 bis 200, mehr bevorzugt von 80 bis 200, noch mehr bevorzugt von 120 bis 200 und besonders bevorzugt von 120 bis 160 Wischtüchern enthält.

16. Verwendung eines Wischtuches nach einem der Ansprüche 1 bis 14 zur Reinigung und/oder Desinfektion einer unbelebten Oberfläche.

17. Verfahren zur Herstellung eines Wischtuches nach einem der Ansprüche 1 bis 14, wobei das Verfahren die folgenden Schritte umfasst:
a. Bereitstellen eines Substrats umfassend Lyocell-Fasern,
b. Bereitstellen eines flüssigen Desinfektionsmittels umfassend die Komponenten:
i. 20 bis 50 Gew.-% mindestens eines einwertigen Alkohols mit 2 bis 4 Kohlenstoffatomen,
ii. mindestens eine weitere antimikrobiell wirksame Substanz,
iii. optional mindestens einen Hilfsstoff,
iv. Wasser,
c. Tränken des Substrats mit dem Desinfektionsmittel, wobei sich der pH-Wert des Desinfektionsmittels bevorzugt um weniger als 10 %, insbesondere um weniger als 5 % ändert.

18. Wischtuch für die Flächendesinfektion umfassend ein Substrat und ein flüssiges Desinfektionsmittel,
**dadurch gekennzeichnet, dass**
- das Substrat Lyocell-Fasern umfasst und
- das Desinfektionsmittel die folgenden Komponenten umfasst:
i. 20 bis 50 Gew.-% mindestens eines einwertigen Alkohols mit 2 bis 4 Kohlenstoffatomen,
ii. optional eine weitere antimikrobiell wirksame Substanz,
iii. optional mindestens einen Hilfsstoff,
iv. Wasser,
wobei das Wischtuch so ausgebildet ist, dass das aus ihm ausgepresste Desinfektionsmittel einen Reduktionsfaktor von mindestens 2, bevorzugt von mindestens 3 und besonders bevorzugt von mindestens 4 gegen Adenovirus aufweist.
